# EUROPÄISCHE PATENTSCHRIFT

(11) **EP 1 321 116 B1**
(45) Veröffentlichungstag und Bekanntmachung des Hinweises auf die Patenterteilung: **04.08.2004**
(21) Anmeldenummer: 01129441.0
(22) Anmeldetag: 10.12.2001
(51) Int. Cl.: A61F 2/46

(54) **Einsetzinstrument für Schlittenprothesen**
Insertion instrument for femoral sled prosthesis
Instrument d'insertion de prothèse femorale à glissement

(43) Veröffentlichungstag der Anmeldung: 25.06.2003
(73) Patentinhaber: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll & Partner

(56) Entgegenhaltungen:
- EP-A- 1 099 430
- US-A- 3 949 428

## Beschreibung

Das menschliche Kniegelenk enthält zwei tibio-femorale Gelenkflächenpaare, nämlich ein mediales und ein laterales Gelenkflächenpaar, die jeweils von einem femoralen Kondylus und einer damit zusammenwirkenden, schalenförmigen tibialen bzw. meniskalen Gelenkfläche gebildet sind. In der prothetischen Nachbildung werden die femoralen Gleitflächen entweder von einem einstückigen Prothesenteil oder von zwei gesonderten Prothesenteilen gebildet. Die letzteren bezeichnet man als Schlittenprothesen. Damit die femoralen und tibialen Gelenkgleitflächen richtig zusammenspielen können, muß ihr seitlicher Abstand gleich sein. Das bedeutet bei Verwendung von Schlittenprothesen, daß deren Abstand genau eingehalten werden muß. Zu diesem Zweck sind Einsetzinstrumente bekannt (EP-A-1 099 430), bei denen an einer Tragplatte des Instruments zwei Klemmen angeordnet sind, die je zur Aufnahme einer Schlittenprothese bestimmt sind. Der Abstand dieser Klemmen ist entsprechend den Vorgaben des zugehörigen tibialen _{P}rothesenteils verstellbar. Die Klemmbacken der Klemmen wirken jeweils mit den Seitenrändern der Schlittenprothesen zusammen. Da die Kraftübertragung an dieser Stelle begrenzt ist, ist in jeder Klemme auch noch eine höhenverstellbare Gleitflächenstütze vorgesehen, die einen wesentlichen Teil der vom Instrument bei der Implantation auf die Prothesenteile zu übertragenden Kraft übernimmt.

Bei dem bekannten Gerät ist je eine Klemmbacke der Klemmen unverstellbar mit der Tragplatte verbunden und trägt sie die seitenverstellbare zweite Klemmbacke. Wenn man den Abstand der Klemmen ändern will, so wählt man eine andere Tragplatte oder andere Klemmen aus. Es müssen also unterschiedliche Tragplatten oder Klemmen vorrätig gehalten werden. Dies ist aufwendig. Auch ist es schwierig, intraoperativ einen anderen Klemmenabstand zu wählen als denjenigen, der bei der Operationsvorbereitung ausgewählt wurde. Die Anordnung der Gleitflächenstützen an den Klemmen ist nachteilig, weil ihre Anordnung kompliziert und ihre Zugänglichkeit erschwert ist.

Bei einem anderen bekannten Einsetzgerät für Schlittenprothesen (US-A-3 949 428) sind die Klemmen in sich nicht verstellbar. Statt dessen weisen die Klemmbacken Führungsschienen auf, die mit entsprechenden Nuten der Schlittenprothesen zusammenwirken. Damit sie von den Schlittenprothesen nach deren Implantation wieder gelöst werden können, müssen die Nuten an den Schlittenprothesen am Ende offen sein. Da sie nicht in den Gleitflächen selbst münden dürfen, ist diese Lösung bei heutzutage üblichen Schlittenprothesen nicht anwendbar. Der Abstand der Klemmen voneinander ist einstellbar, indem sie verschiebbar an einem Haltegestell angeordnet sind und je eine Arretierschraube aufweisen, mittels derer ihre jeweils gewählte Stellung fixiert werden kann. Es handelt sich um eine sehr umständliche Anordnung, die eine Änderung der Abstandsverhältnisse unter Operationsbedingungen kaum möglich macht.

Ausgehend von dem eingangs geschilderten Stand der Technik liegt der Erfindung die Aufgabe zugrunde, die Konstruktion zu vereinfachen und leichter bedienbar zu machen. Dies gelingt mit Hilfe der Merkmale des Anspruchs 1 und vorzugsweise der Merkmale der Unteransprüche.

Demnach ist das erfindungsgemäße Instrument dadurch gekennzeichnet, daß die Tragplatte eine Gleitführung mit Verstellspindel für wenigstens eine der beiden Klemmen aufweist und die Gleitflächenstützen auch fest an der Tragplatte in einer dem Verstellbereich entsprechenden Breite angeordnet sind. Durch Betätigung der Verstellspindel kann der Abstand der Klemmen einfach verstellt werden. Ferner erleichtert dieses Merkmal die Lösung des Instruments von den implantierten Prothesen. Dafür werden nicht nur die Klemmen erweitert, sondern es können durch die Abstandsverstellung auch diejenigen Klemmbacken, die bei der Erweiterung der Klemmen unverändert bleiben, aus dem Eingriff mit den Prothesenteilen gelöst werden. Was die Anordnung der Gleitflächenstützen angeht, so hat die Erfindung erkannt, daß diese nicht unbedingt genau dort angeordnet sein müssen, wo die Schlittenprothesen sich befinden. Das würde bedeuten, daß ihr Abstand zusammen mit dem der Klemmen geändert werden müßte. Aus diesem Grunde befanden sich die Gleitflächenstützen bei dem eingangs erläuterten, bekannten Gerät an den Klemmen selbst. Die Erfindung hat erkannt, daß die Konstruktion wesentlich vereinfacht werden kann, wenn die Gleitflächenstützen an der Tragplatte mit konstantem Abstand angeordnet werden. Dabei ist lediglich erforderlich, daß ihre Breite so groß ist, daß sie bei jeder Abstandseinstellung noch hinreichend mit den Prothesenteilen zusammenwirken können. Die Anordnung der Gleitflächenstützen getrennt von den Klemmen hat die Konsequenz, daß die in den Klemmen befindlichen Prothesenhalterungen gegenüber den Gleitflächenstützen in anterior-posteriorer Richtungversetzt angeordnet sind. Dies ist deshalb vorteilhaft, weil dadurch die Prothesenteile sicherer von dem Instrument gefaßt und beträchtliche Kräfte auf die Prothesen übertragen werden können, ohne daß deren Lageveränderung befürchtete werden muß.

Um den Abstand der Klemmen voneinander verstellen zu können, reicht es aus, wenn die Tragplatte nur eine Gleitführung mit Verstellspindel für eine der beiden Klemmen aufweist. Daraus kann sich aber je nach eingestelltem Abstand eine unsymmetrische Anordnung der Klemmen ergeben. Zweckmäßiger ist es deshalb, wenn beide Klemmen in Gleitführungen der Tragplatte gehalten sind und die Verstellspindel gegenläufig auf beide Klemmen wirkt.

Die Klemmen bestehen zweckmäßigerweise aus einer unmittelbar mit der Tragplatte verbundenen Klemmbacke und einer von dieser gehaltenen und ihr gegenüber verstellbaren Klemmbacke. Jedoch soll der Fall nicht ausgeschlossen sein, in welchem jede Klemmbacke aus einem von der Tragplatte gehaltenen Basisteil und zwei gegenüber dem Basisteil verstellbaren Klemmbacken besteht.

Nach einem wichtigen Merkmal der Erfindung sind die Gleitflächenstützen dem in anterior-posteriorer Richtung verlaufenden Gleitflächenabschnitt zugeordnet, weil das Ansetzen der Schlittenprothesen an das Femur im allgemeinen in einer nach distal verlaufenden Richtung erfolgt und somit die Gleitflächenstützen die Ansetzkraft im wesentlichen übernehmen können. Auch verlaufen auf der proximalen Seite der Prothesen gegebenenfalls vorhandene Verankerungszapfen im allgemeinen in dieser Richtung. Sollten jedoch die Verankerungszapfen in anderer Richtung verlaufen, so kann es zweckmäßiger sein, die Gleitflächenstützen so anzuordnen, daß sie in dieser anderen Richtung wirken.

Die Gleitflächenstützen sollten höhenverstellbar sein, damit sie an unterschiedliche Prothesenformen angepaßt werden können.

Die Erfindung wird im folgenden näher unter Bezugnahme auf die Zeichen erläutert, die ein vorteilhaftes Ausführungsbeispiel veranschaulicht. Es zeigen:

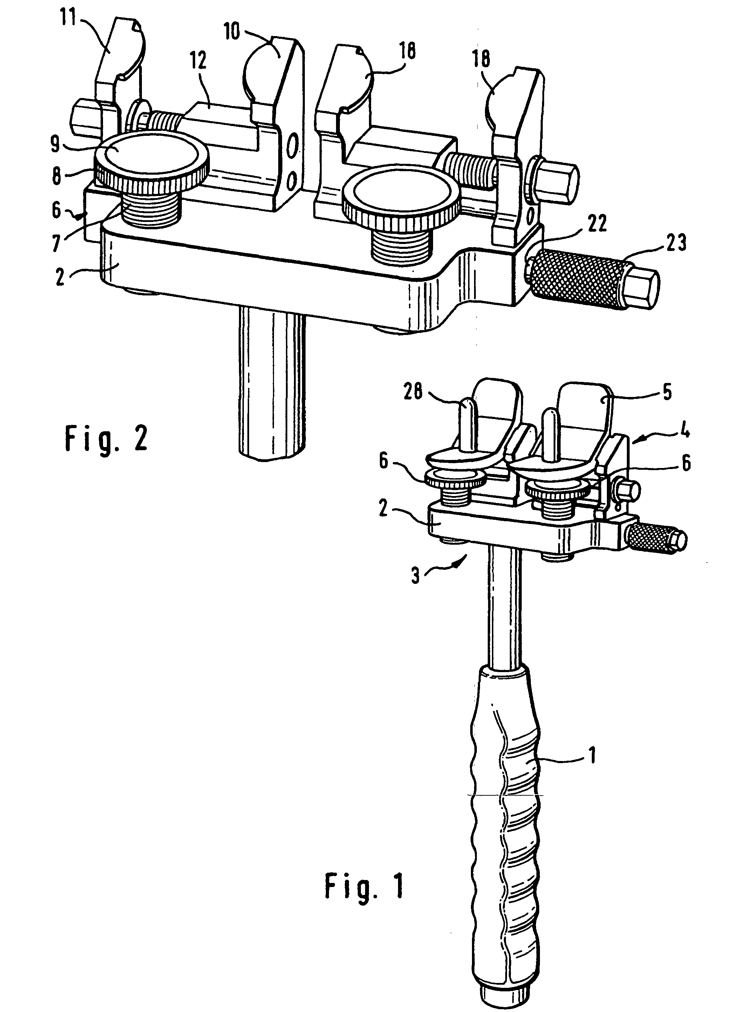

- Fig. 1: eine Gesamtansicht des Instruments mit angesetzten Schlittenprothesen,
- Fig. 2: den vorderen Teil des Instruments in größerem Maßstab ohne die Schlittenprothesen,
- Fig. 3: eine Stirnansicht des Instruments mit angesetzten Schlittenprothesen,
- Fig. 4: eine Seitenansicht des vorderen Teils des Instruments,

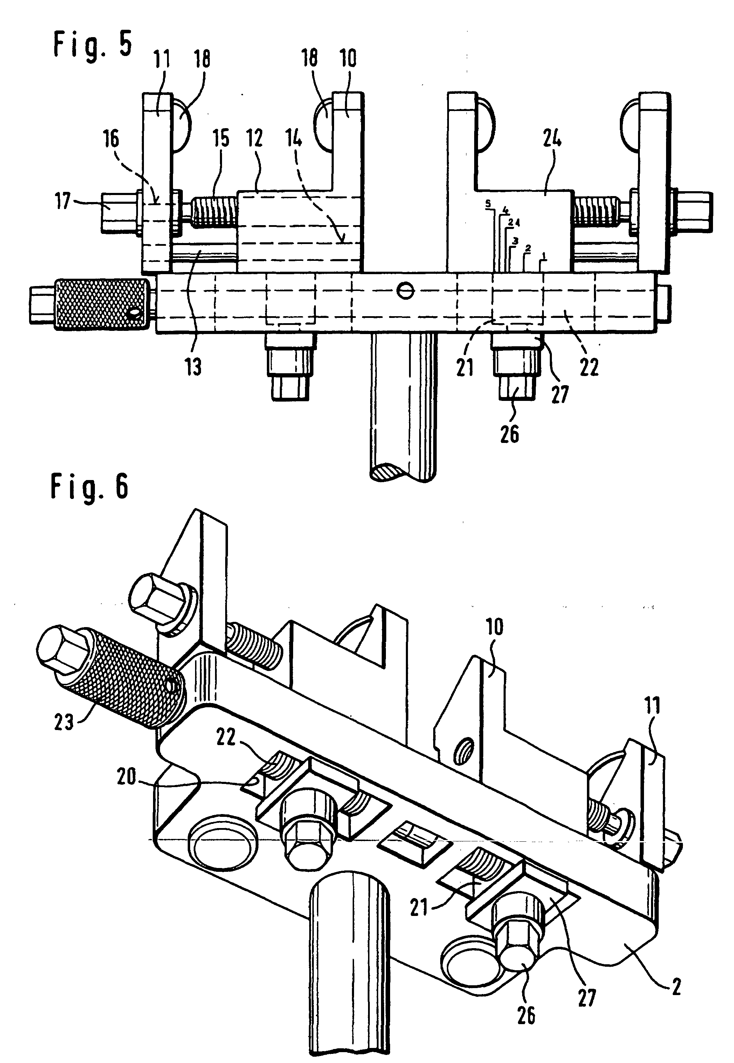

- Fig. 5: eine Rückansicht des vorderen Teils des Instruments und
- Fig. 6: eine Ansicht des vorderen Teils des Instruments schräg von hinten.

An einem Stiel mit Griff 1 ist die Tragplatte 2 des vorderen Instrumententeils 3 befestigt. Die Tragplatte trägt zwei Klemmen 4 für zwei Schlittenprothesen 5 sowie zwei Gleitflächenstützen 6.

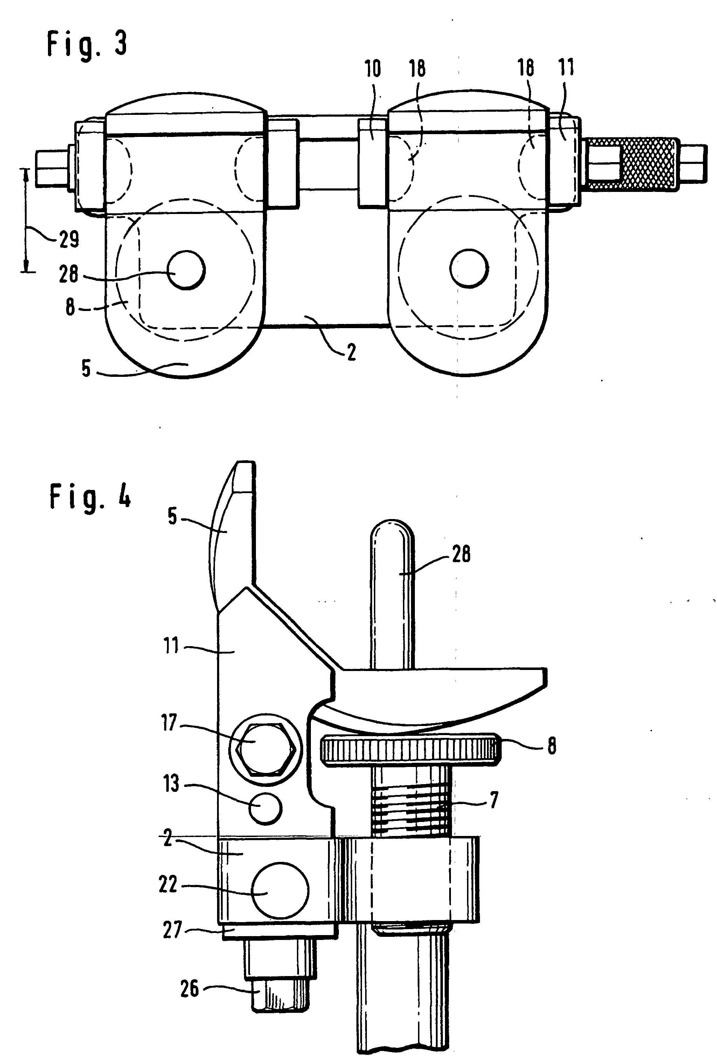

Die Gleitflächenstützen 6 bestehen aus einem in der Tragplatte gehaltenen Gewindeschaft 7 und einem Kopf 8, der ein Schlagpolster 9 aus steifelastischem Kunststoff enthält, beispielsweise Polyethylen. Die Oberfläche des Kopfs 8 ist im wesentlichen eben. Durch Verdrehung der Gleitflächenstütze mittels einer am Kopf 8 vorgesehenen Rändelung läßt sie sich so einstellen, daß ihre Oberseite stützend an der Gleitfläche des in der zugehörigen Klemme 4 befindlichen Prothesenteils anliegt, wie Fig.4 dies zeigt.

Jede Klemme 4 umfaßt eine erste Klemmbacke 10 und eine zweite Klemmbacke 11. Der Fuß 12 der ersten Klemmbacke 10 wird von der Halteplatte 2 in später zu erläuternder Weise gehalten. Die zweite Klemmbacke 11 ist parallel verschiebbar vom Fuß 12 der ersten Klemmbacke 10 gehalten. Zu diesem Zweck ist an der zweiten Klemmbacke 11 eine Führungsstange 13 starr angeordnet, die in einer Bohrung 14 des Fußes 12 geführt ist. Außerdem ist eine Gewindespindel 15 vorgesehen, die in dem Fuß 12 verschraubbar ist und deren Ende in einer Bohrung 16 der zweiten Klemmbacke gelagert ist. Durch Verdrehen des Kopfs 17 der Spindel 15 kann somit der Abstand der Klemmbacken 10, 11 eingestellt werden.

Beide Klemmbacken 10, 11 tragen einander gegenüberliegende, langgestreckte Vorsprünge 18. Die Schlittenprothesen 5 enthalten an entsprechender Stelle passende Schlitze, in die die Vorsprünge 18 eingreifen, wenn die Prothesen in die Klemmen eingesetzt sind. Dadurch werden die Prothesen gegenüber dem Instrument positioniert.

Die Tragplatte 2 enthält unter den Füßen 12 je einen in Querrichtung des Instruments (parallel zu der Führungsstange 13) verlaufender Führungsschlitz 20, in welchem ein passender Gleitstein 21 geführt ist, der fest mit dem Fuß 12 verbunden ist. Eine durchgehende Spindel 22 liegt längs in den beiden Führungsschlitzen 20. Sie ist in der Tragplatte 2 drehbar, axial unbeweglich gelagert und mittels eines gerändelten Kopfs 23 drehbar. Im Bereich jedes der beiden Schlitze 20 weist sie einen Gewindeabschnitt auf. Die Gewindeabschnitte liegen in passenden Gewindebohrungen der Gleitsteine 21 und sind gegenläufig. Wird der Kopf 23 der Spindel gedreht, so werden, je nach Drehrichtung, die Klemmen 4 einander angenähert oder voneinander entfernt. Ihr Abstand und damit der Abstand der von ihnen gehaltenen Schlittenprothesen kann auf diese Weise beliebig eingestellt werden. Zur leichteren Einstellung dient eine Skala 24, die mit einer entsprechenden Markierung an der Tragplatte zusammenwirkt.

Die Gleitsteine 21 sind an der Unterseite der Tragplatte 2 über Stellschrauben 26 mit Querjochen 27 verbunden. Diese können in der gewünschten Einstellung der Klemmen angezogen werden, um die Lage der Klemmen zu fixieren. Wenn sie nur schwach angezogen werden, bleiben die Klemmen 4 an der Tragplatte 2 verschiebbar, aber es kann ihr Spiel gegenüber der Tragplatte eingestellt werden.

Das Instrument wird in folgender Weise benutzt. Nach Auswahl der geeigneten Größe der Schlittenprothesen 5 werden diese in die Klemmen 4 des Instruments eingesetzt und werden die Gleitflächenstützen 6 so eingestellt, daß sie an den Gleitflächen der Prothesen stützend anliegen. Entsprechend den anatomischen Vorgaben bzw. entsprechend den Maßen einer gegebenenfalls in Verbindung mit den Schlittenprothesen zu verwendenden tibialen Prothese wird der Abstand der Schlittenprothesen voneinander mittels der Spindel 22 eingestellt. Die Prothesen werden dann in bekannter Weise implantiert. Für die dabei aufzuwendende Kraftübertragung ist es hilfreich, daß der Griff 1 mit den Gleitflächenstützen 6 und den Verankerungsstiften 28 der Prothesen 4 (siehe Fig. 4) im wesentlichen fluchtet. Für die Kraftübertragung auf die Prothesen und für deren Lagesicherung ist es ferner vorteilhaft, daß die Gleitflächenstützen 6 und die an den Klemmen 4 vorgesehenen Vorsprünge 18 in anterior-posteriorer Richtung einen verhältnismäßig großen Abstand 29 voneinander haben, der darin begründet liegt, daß die Gleitflächenstützen 6 auf den in anterior-posteriorer Richtung verlaufenden Teil der Gleitflächen wirken, während die Vorsprünge 18 mit einem Abschnitt der Prothesen zusammenwirken, dessen Gleitfläche im Mittel um wenigstens 30° (im dargestellten Beispiel 45°) gegenüber der anterior-posterioren Richtung geneigt ist.

Nach dem Ansetzen der Prothesen an das Femur werden die Klemmen 4 so weit geöffnet, daß der Abstand zwischen den Vorsprüngen 18 größer ist als die Breite der Prothesen. Da die an den ersten Klemmbacken 10 befindlichen Vorsprünge danach noch in die zugehörigen Nuten der Prothesen eingreifen, werden die Klemmen einander durch Betätigung des Spindelkopfs 23 angenähert, bis das Instrument von den Prothesen abgenommen werden kann.

## Patentansprüche

1. Einsetzinstrument für ein Paar von Schlittenprothesen (5), das an einer Tragplatte (2) zur Aufnahme je einer Schlittenprothese (5) zwei Klemmen (4) mit wählbarem Abstand trägt, deren Klemmbacken (10, 11) mit den Seitenrändern der Schlittenprothesen (5) zusammenwirken, für deren Unterstützung im Bereich der Gleitflächen zwei höhenverstellbare Gleitflächenstützen (6) vorgesehen sind, **dadurch gekennzeichnet, daß** die Tragplatte (2) eine Gleitführung (20) mit Verstellspindel (22) für wenigstens eine der beiden Klemmen (4) aufweist und Gleitflächenstützen (6) ortsfest an der Tragplatte (2) angeordnet sind und eine dem Verstellbereich der Klemmen (4) entsprechende Breite aufweisen.

2. Instrument nach Anspruch 1, **dadurch gekennzeichnet, daß** nur eine Verstellspindel (22) mit gegenläufigem Gewinde für beide Klemmen (4) vorgesehen ist.

3. Instrument nach Anspruch 1 oder 2, **dadurch gekennzeichnet, daß** die Klemmen (4) eine unmittelbar mit der Tragplatte (2) verbundene Klemmbacke (10) und eine von dieser gehaltene und ihr gegenüber verstellbare Klemmbacke (11) umfassen.

4. Instrument nach einem der Ansprüche 1 bis 3, **dadurch gekennzeichnet, daß** die Gleitflächenstützen (6) dem in anterior-posteriorer Richtung verlaufenden Gleitflächenabschnitt zugeordnet sind.

5. Instrument nach einem der Ansprüche 1 bis 4, **dadurch gekennzeichnet, daß** die Gleitflächenstützen (6) in der Richtung wirken, die der Richtung eines an den Schlittenprothesen (4) vorgesehenen Verankerungszapfens (2,9) entspricht.

## Claims

1. An insertion instrument for a pair of sliding prostheses (5), said instrument having, on a supporting plate (2), two clamps (4) which are each intended to receive a sliding prosthesis (5) and whose spacing is adjustable, their clamping jaws (10, 11) interacting with the side edges of the sliding prostheses (5), and two vertically adjustable slide-surface supports (,6) being provided to support the sliding prostheses (5) in the area of the slide surfaces, wherein the supporting plate (2) has a slide guide (20) with adjustment spindle (22) for at least one of the two clamps (4), and slide-surface supports (6) are arranged in a fixed position on the supporting plate (2) and have a width which corresponds to the adjustment range of the clamps (4).

2. The instrument as claimed in claim 1, wherein only one adjustment spindle (22) with opposite threads is provided for both clamps (4).

3. The instrument as claimed in claim 1 or 2, wherein the clamps (4) comprise a clamping jaw (10) connected directly to the supporting plate (2), and a clamping jaw (11) held by the clamping jaw (10) and adjustable relative thereto.

4. The instrument as claimed in one of claims 1 through 3, wherein the slide-surface supports (6) are assigned to the slide-surface portion extending in the anterior-posterior direction.

5. The instrument as claimed in one of claims 1 through 4, wherein the slide-surface supports (6) act in the direction corresponding to the direction of an anchoring pin (28) provided on the sliding prostheses (5).

## Revendications

1. Instrument d'insertion pour une paire de prothèses à charnière (5), qui porte, sur une plaque de support (2), deux étaux (4) à écartement sélectionnable, destinés à recevoir chacun une prothèse à charnière (5), dont les mâchoires (10, 11) coopèrent avec les bords latéraux des prothèses à charnière (5), deux appuis (6) pour surfaces de glissement, réglables en hauteur, étant prévus pour les soutenir dans la zone des surfaces de glissement, **caractérisé en ce que** la plaque de support (2) comporte une glissière (20) avec vis de réglage (22) pour au moins l'un des deux étaux (4) et des appuis (6) pour surfaces de glissement sont disposés en un emplacement fixe sur la plaque de support (2) et présentent une largeur correspondant à la zone de réglage des étaux (4).

2. Instrument selon la revendication 1, **caractérisé en ce qu'**il n'est prévu qu'une vis de réglage (22) à filetage en sens contraire pour les deux étaux (4).

3. Instrument selon la revendication 1 ou 2, **caractérisé en ce que** les étaux (4) comprennent une mâchoire (10) directement reliée à la plaque de support (2) et une mâchoire (11) maintenue par celle-ci et réglable par rapport à celle-ci.

4. Instrument selon l'une des revendications 1 à 3, **caractérisé en ce que** les appuis (6) pour surfaces de glissement sont associés au tronçon de surface de glissement s'étendant dans la direction antérieure-postérieure.

5. Instrument selon l'une des revendications 1 à 4, **caractérisé en ce que** les appuis (6) pour surfaces de glissement agissent dans la direction qui correspond à la direction d'une tige d'ancrage (2, 9) prévue sur les prothèses à charnière.
